# EUROPEAN PATENT APPLICATION

(11) **EP 3 176 169 A1**
(43) Date of publication of application: **07.06.2017**
(21) Application number: 16200797.5
(22) Date of filing: 31.01.2007
(51) Int. Cl.: C07D 471/04, A61K 31/437, A61P 31/18, A61P 35/00, C12N 15/29, C12N 15/09, C12N 15/10

(54) **POSITIVELY CHARGED WATER-SOLUBLE PRODRUGS OF 1H-IMIDAZO[4,5-C]QUINOLIN-4-AMINES AND RELATED COMPOUNDS WITH VERY HIGH SKIN PENETRATION RATES**

(62) Divisional of application: 07705747.9
(71) Applicant: Yu, Chongxi, Kensington, MD 20895 (US)
(72) Inventor: Yu, Chongxi, Kensington, MD 20895 (US)
(74) Representative: Finnegan Europe LLP

(57) **Abstract**

The novel positively charged pro-drugs of 1H-imidazo[4, 5-c]quinolin-4-amines and related compounds in the general formula (1) 'Structure 1' or formula (2) 'Structure 2' were designed and synthesized. The compounds of the general formula (1) 'Structure 1' or formula (2) 'Structure 2' indicated above can be prepared from 1H-imidazo[4, 5-c]quinolin-4-amines and related compounds, by reaction with suitable acetic anhydride or acetic chloride. The positively charged amino groups of these pro-drugs not only largely increases the solubility of the drugs in water, but also bonds to the negative charge on the phosphate head group of membranes. This bonding will disturb the membrane a little bit and may make some room for the lipophilic portion of the prodrug. When the molecules of membrane move, the membrane may 'crack' a little bit due to the bonding of the prodrug. This will let the prodrug insert into the membrane. At pH 7.4, only about 99% of the amino group is protonated. When the amino group is not protonated, the bonding between the amino group of the prodrug and the phosphate head group of the membrane will disassociate, and the prodrug will enter the membrane completely. When the amino group of the prodrug flips to the other side of the membrane and thus becomes protonated, then the prodrug is pulled into the cytosol, a semi-liquid concentrated aqueous solution or suspension. The results suggest that the pro-drugs diffuse through human skin -25 times faster than do 1H-imidazo[4, 5-c]quinolin-4-amines and related compounds. In plasma, more than 90% of these pro-drugs can change back to the parent drugs in a few minutes. The prodrugs can be used medicinally in treating any 1H-imidazo[4,5-c]quinolin-4-amines and related compounds-treatable conditions in humans or animals. The prodrugs can be administered transdermally for any kind of medical treatments and avoid most of the side effects of 1H-imidazo[4,5-c] quinolin-4-amines and related compounds. Controlled transdermal administration systems of the prodrug enables 1H-imidazo[4, 5-c]quinolin-4-amines and related compounds to reach constantly optimal therapeutic blood levels to increase effectiveness and reduce the side effects of 1H-imidazo[4, 5-c]quinolin-4-amines and related compounds.

## Description

### Technical Field

The present invention relates to the preparations of positively charged and watersoluble pro-drugs of 1H-imidazo[4,5-c]quinolin-4-amines and related compounds and their medicinal use in treating any 1H-imidazo[4,5-c]quinolin-4-amines and related compounds-treatable conditions in humans or animals. More specifically, the present invention is to enable quick skin penetration of 1H-imidazo[4,5-c]quinolin-4-amines and related compounds.

### Background Art

1H-Imidazo[4,5-c]quinolin-4-amines and related compounds are disclosed in U.S. Pat. No. 4,689,338 and described therein as antiviral agents and as an interferon inducer. The anti-herpes activity of 1-Isobutyl-1H-imidazo[4,5-c]quinolin-4-amine and related compounds relative to primary lesions caused by the herpes simplex virus is demonstrated by Gerster, J.F. (U.S. Pat. No. 4,689,338) using the method described generally by Kern, et al. (Antimicrob. Agents Chemother. 14, 817-823, 1978).

A variety of formulations for topical administration of this compound is also described. U.S. Pat. No. 4,751,087, 4,411,893, 4,722,941, 4,746,515, and 5,736,553 disclosed the use of a combination of ethyl cleats and glyceryl monolaurate, N,N-dimethyldodecylamine-N-oxide, glyceryl monolaurate, and fatty acid as skin penetration enhancers to enhance the Transdermal flux of drugs. Aldara (1-Isobutyl-1H-imidazo[4,5-c]quinolin-4-amine) cream has been developed by 3M for the treatment of actinic keratosis, superficial basal cell carcinoma, and external genital and perianal warts.

### Disclosure of Invention

### Technical Problem

Aldara (1-Isobutyl-1H-imidazo[4,5-c]quinolin-4-amine) cream has been developed by 3M for the treatment of actinic keratosis, superficial basal cell carcinoma, and external genital and perianal warts.

Unfortunately, 1-Isobutyl-1H-imidazo[4,5-c]quinolin-4-amine and related compounds have very low solubility in water and organic solvents so their skin penetration rates are very low. The cream compositions will keep the drug on the skin for a very long time in very high concentration and cause many side effects that include redness, swelling, sores, blisters, or ulcers, skin that becomes hard or thickened, skin peeling, scabbing and crusting, itching, burning and changes in skin color that do not always go away. Another problem is that they cannot penetrate skin deep enough to treat cancers except for superficial basal cell carcinoma.

### Technical Solution

This invention relates to the preparation of novel positively charged pro-drugs of 1H-imidazo[4,5-c]quinolin-4-amines and related compounds and their medicinal use. The pro-drugs of 1H-imidazo[4,5-c]quinolin-4-amines and related compounds have the general formula (1) 'Structure 1' or formula (2) 'Structure 2': 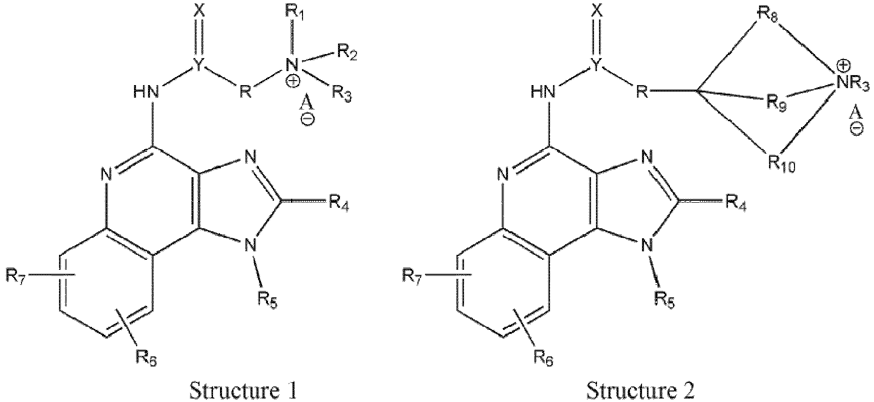 wherein, R represents a branched or straight chain, -(CH₂)ₙ-, wherein n=0, 1, 2, 3, 4, 5, 6, 7, 8, 9, 10 ..., in -(CH)₂)ₙ-, any CH₂ may be replaced with O, S, CH=CH, C≡C, CR₁₁ R₁₂, aryl or heteroaryl residues, or other ring systems; R₁ and R₂ taken alone are same or different and are H, one of any alkyl, alkyloxyl, alkenyl, perfluoroalkyl, alkyl halide or alkynyl residues having 1 to 12 carbon atoms, aryl or heteroaryl moieties or taken together are -(CH₂)ₘ-, wherein m=2, 3, 4, 5, 6, 7, 8, 9, 10,..., and any CH₂ may be replaced with O, S, CH=CH, C≡C, CR₁₁R₁₂, aryl or heteroaryl moieties, or other ring moieties; R₃ represents H, one of any alkyl, alkyloxy, alkenyl, perfluoroalkyl, alkyl halide, or alkynyl residues having 1 to 12 carbon atoms, aryl or heteroaryl moieties, wherein, any CH may be replaced with O, S, CH=CH, C≡C, CR₁₁R₁₂, aryl or heteroaryl moieties, or other ring moieties; X represents O, NH, or S; Y represents C, S=O, or P-OR₁₁; R₄ is selected from the group consisting of alkyl of 1 to about 10 carbon atoms, hydroxylalkyl of 1 to about 10 carbon atoms, acyloxyalkyl wherein the acyloxy moiety is alkanoyloxy of 2 to about 5 carbon atoms or benzoyloxy, and the alkyl moiety contains 1 to about 8 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to about 5 carbon atoms, alkoxy of 1 to about 5 carbon atoms and halogen, with the proviso that if said benzene ring is substituted by 2 of said moieties, then said moieties together contain no more than 8 carbon atoms; R₅ is selected from the group consisting of hydrogen, alkyl of 1 to about 10 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to about 5 carbon atoms, alkoxy of 1 to about 5 carbon atoms and halogen, with the proviso that if said benzene ring is substituted by 2 of said moieties, then said moieties together contain no more than 8 carbon atoms; R₆ is selected from the group consisting of hydrogen, alkyl of 1 to about 5 carbon atoms, and alkoxy of 1 to about 5 carbon atoms and halogen; R₇ is selected from the group consisting of hydrogen, alkyl of 1 to about 5 carbon atoms, and alkoxy of 1 to about 5 carbon atoms and halogen; R₈ represents a branched or straight chain, -(CH₂)ₐ-, wherein a=0, 1, 2, 3, 4, 5, 6, 7, 8, 9, 10 ..., in -(CH₂)ₐ-, any CH₂ may be replaced with O, S, CH=CH, C≡C, CR₁₁R₁₂, aryl or heteroaryl residues, or other ring systems; R₉ represents a branched or straight chain, -(CH₂)_{b}-, wherein b=0, 1, 2, 3, 4, 5, 6, 7, 8, 9, 10 ..., in -(CH₂)_{b}-, any CH₂ may be replaced with O, S, CH=CH, C≡C, CR₁₁R₁₂, aryl or heteroaryl residues, or other ring systems; R₁₀ represents a branched or straight chain, -(CH₂)_{c}-, wherein c=0, 1, 2, 3, 4, 5, 6, 7, 8, 9, 10 ..., in -(CH₂)_{c}-, any CH₂ may be replaced with O, S, CH=CH, C=C, CR₁₁R₁₂, aryl or heteroaryl residues, or other ring systems; R₁₁ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁R₂, aryl or heteroaryl moieties, or other ring moieties; R ₁₂ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁R₂, aryl or heteroaryl moieties, or other ring moieties; A⁻ represents Cl⁻, Br⁻, F⁻, I⁻, AcO⁻, citrate, or any negative ions. All R, R₁, R₂, R₃, R₄, -(CH₂)ₙ-, -(CH₂)ₘ-, -(CH₂)ₐ-, -(CH₂)_{b}-, or -(CH₂)_{c}- groups are branched or straight chains and may include C, H, O, S, P, or N atoms and may have single, double, and triple bonds.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. The membranes are bilayers, with the hydrophilic head groups facing outward into the aqueous regions on either side. Very hydrophilic drugs cannot pass the hydrophobic layer of a membrane and very hydrophobic drugs will stay in the hydrophobic layer as part of the membrane due to their similarities and cannot efficiently enter the cytosol on the inside.

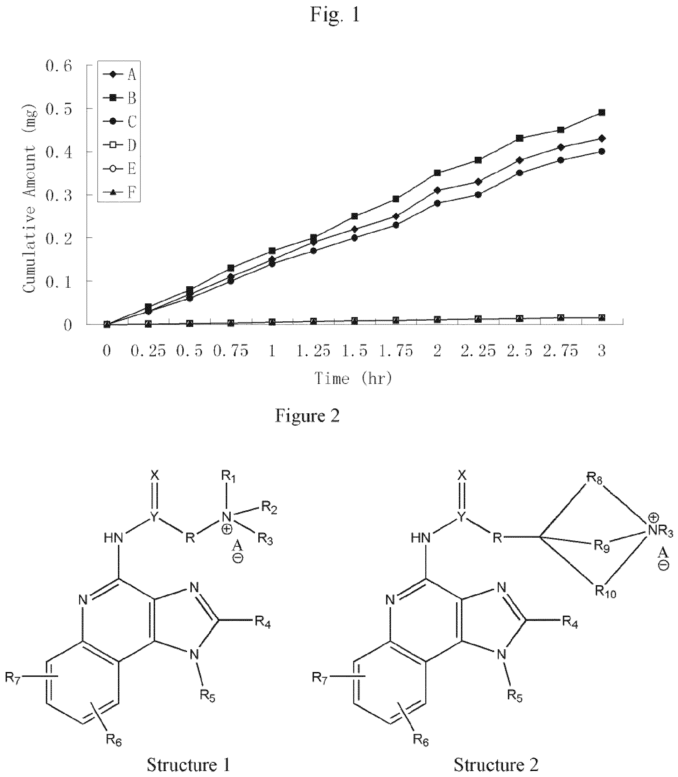

The goal of this invention is to make 1H-Imidazo[4,5-c]quinolin-4-amines and related compounds administrable transdermally (topical application) by increasing their solubility in the moisture available on the skin surface and increasing their penetration rate through the membrane and skin barrier. These novel pro-drugs of 1H-Imidazo[4,5-c]quinolin-4-amines and related compounds have two structural features in common: they have a lipophilic portion and a primary, secondary, or tertiary amine group that exists in the protonated form (hydrophilic part, their pKa are 9.5-10.7) at physiological pH [The aromatic amines of 1H-Imidazo[4,5-c] quinolin-4-amines are very weak bases (pKa is 6.5-6.7) and only a small part of them exists in the protonated form at physiological pH]. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water. In many instances, the lowest or rate-limiting step in the sequence is the dissolution of the drug. 1H-Imidazo[4,5-c]quinolin-4-amines and related compounds have a very low solubility in the moisture available on the skin surface, and they will not pass across the barrier of skin in a molecular form ef ficiently. Even if they enter the membranes of the skin, they cannot efficiently enter the cytosol, a semi-liquid concentrated aqueous solution or suspension on the inside of the cell (due to their low water solubility). When these new pro-drugs are administered transdermally in a dosage form such as a solution, spray, lotion, ointment, emulsion or gel, they will dissolve in the moisture available on the skin surface immediately. The positive charge on the amino groups of these pro-drugs will bond to the negative charge on the phosphate head group of a membrane [the bonding between the positive charge on the aromatic amines of 1H-imidazo[4,5-c]quinolin-4-amines and the negative charge on the phosphate head group of a membrane is very weak due to the weak basity (pKa is about 6.5-6.7) and hindered position (the amino groups connected with aromatic ring directly)], thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol. When the prodrugs are outside the skin membranes, they are only prodrugs, which may be without biological activity; because of that and due to the short stay on the outside of the membrane, the prodrugs will not cause redness, swelling, sores, blisters, or ulcers in the skin, hardened or thickened skin, skin peeling, scabbing and crusting, itching, burning or changes in skin color. The penetration rates of these prodrugs through human skin were measured in vitro by using modified Franz cells, which were isolated from human skin tissue (360-400 µm thick) of the anterior and posterior thigh areas. The receiving fluid consisted of 2 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 3% solution of some of the prodrugs of 1H-imidazo[4,5-c]quinolin-4-amines or a 3% suspension of 1H-imidazo[4,5-c]quinolin-4-amines in 0.5mL of a mixture of ethanol and pH 7.4-phosphate buffer (0.2M) (v/v, 70/30) are shown in Figure 1. Apparent flux values of 0.15 mg, 0.13 mg, 0.16 mg, 0.005 mg, 0.005 mg, and 0.005 mg/cm²/h were calculated for sarcosine 1-isobutyl-1H-imidazo[4,5-c]quinolin-4-amide hydrochloride, sarcosine 1-butyl-1H-imidazo[4,5-c]quinolin-4-amide hydrochloride, sarcosine 1-benzyl-1H-imidazo[4,5-c]quinolin-4-amide hydrochloride, 1-isobutyl-1H-imidazo[4,5-c]quinolin-4-amide hydrochloride, 1-butyl-1H-imidazo[4, 5-c]quinolin-4-amine hydrochloride, and 1-benzyl-1H-imidazo[4,5-c] quinolin-4-amine hydrochloride respectively diffusing through human skin. The pro-drugs diffuse through human skin more than 25 times faster than do 1H-imidazo[4,5-c] quinolin-4-amines. The results suggest that the positive charge on the di-alkyaminoethyl group has a very important role in the passage of the drug across the membrane and skin barrier.

Irritative effect or discomfort in the skin of mice of the novel prodrugs was evaluated during a period of 1 week after the topical application of 0.05 ml of 3 % of the respective test drug in pH 7.4 phosphate buffer (0.2 M) to the back of nude mice twice per day. None of any signs of irritative effect or discomfort was observed for sarcosine 1-isobutyl-1H-imidazo[4,5-c]quinolin-4-amide hydrochloride, sarcosine 1-butyl-1H-imidazo[4,5-c]quinolin-4-amide hydrochloride, sarcosine 1-benzyl-1H-imidazo[4,5-c]quinolin-4-amide hydrochloride.

A good prodrug should change back to the parent drug easily. In vitro plasma hydrolysis studies were carried out as the following. 10 mg of the prodrug was dissolved in 0.1 ml of 0.2M pH 7.4 phosphate buffer. 1 ml of human plasma, preheated to 37°C, was added into the mixture. The mixture was kept in a water bath at 37°C. At every 2 min. intervals, 0.2 ml of samples were withdrawn and added to 0.4 ml of methanol to precipitate the plasma protein. The samples were centrifuged for 5 min and analyzed by HPLC. The half lives of hydrolysis are 18 min+/-1 min. for sarcosine 1-isobutyl-1H-imidazo[4,5-c]quinolin-4-amide hydrochloride, 18 min+/-2 min. for sarcosine 1-butyl-1H-imidazo[4,5-c]quinolin-4-amide hydrochloride, 19 min+/-1 min. for sarcosine 1-isobutyl-1H-imidazo[4,5-c]quinolin-4-amide hydrochloride

1H-imidazo[4,5-c]quinolin-4-amines are known antiviral agents that are also known to induce interferon biosynthesis (Gerster, J.F., U.S. Pat. No. 4,689,338). The fact that the compounds are interferon inducers suggests that they may be useful in the treatment of numerous diseases, such as rheumatoid arthritis, genital or other warts, eczema, hepatitis, psoriasis, multiple sclerosis, essential thrombocythaemia, cancers, acquired immunodeficiency syndrome (AIDS) and other viral diseases. Aldara (1-Isobutyl-1H-imidazo[4,5-c]quinolin-4-amine) cream has been developed by 3M for the treatment of actinic keratosis, superficial basal cell carcinoma, and external genital and perianal warts.

For evaluation of antitumor activity of these prodrugs, human breast cancer cells (BCAP-37, 4-5 mm³ of tumor tissue was used in each mouse) were subcutaneously xenografted into nude mice (BALB). After 3 hour, 50 µl of 3 % of sarcosine 1-isobutyl-1H-imidazo[4,5-c]quinolin-4-amide hydrochloride and 1-isobutyl-1H-imidazo[4,5-c]quinolin-4-amide hydrochloride in ethanol/0.2M pH 7.2 phosphate buffer (v/v, 70/30) was topically applied to the human breast cancer cells-implanted area (near the front leg) once per day. After 28 days, the control group (n=7, the average tumor size was 15 ± 2 mm x 13 ± 2 mm) and the groups that were treated with 1-isobutyl-1H-imidazo[4,5-c]quinolin-4-amide hydrochloride group (n=7, the average tumor size was 12 ± 2 mm x 11 ± 2 mm) demonstrated 100% incidence , but none of tumor was seen in the group (n=7) that were treated with sarcosine 1-isobutyl-1H-imidazo[4,5-c]quinolin-4-amide hydrochloride. The average weights of the mice were 22 ± 2 grams for the sarcosine 1-isobutyl-1H-imidazo[4,5-c] quinolin-4-amide hydrochloride treated group, 22 ± 2 grams for 1-isobutyl-1H-imidazo[4,5-c] quinolin-4-amide hydrochloride treated group, and 24 ± 2 grams for the control group. The results also show that the prodrug has very mild side effects.

In the second antitumor experiment, human colon cancer cells (LS174J, 4-5 mm³ of tumor tissue was used in each mouse) were subcutaneously xenografted into nude mice (BALB). After 3 hour, 50 µl of 3 % of sarcosine 1-isobutyl-1H-imidazo[4,5-c] quinolin-4-amide hydrochloride and 1-isobutyl-1H-imidazo[4,5-c]quinolin-4-amide hydrochloride in ethanol/0.2M pH 7.2 phosphate buffer (v/v, 70/30) was topically applied to the human colon cancer cells-implanted area (near the front leg) (twice per day). After 28 days, the control group (n=7, the average tumor size was 20 ± 3 mm x 18 ± 3 mm) and the groups that were treated with 1-isobutyl-1H-imidazo[4,5-c] quinolin-4-amide hydrochloride group (n=7, the average tumor size was 17 ± 2 mm x 15 ± 2 mm) demonstrated 100% incidence, but none of tumor was seen in the group (n=7) that were treated with sarcosine 1-isobutyl-1H-imidazo[4,5-c]quinolin-4-amide hydrochloride. The average weights of the mice are 21 ± 2 grams for the sarcosine 1-isobutyl-1H-imidazo[4,5-c]quinolin-4-amide hydrochloride treated group, 21 ± 2 grams for 1-isobutyt-1H-imidaxo[4,5-c]quinolin-4-amide hydrochloride treated group , and 23 ± 2 grams for the control group.

In the third antitumor experiment, human breast cancer cells (BCAP-37, 3-4 mm³ of tumor tissue was used in each mouse) were subcutaneously xenografted into nude mice (BALB). After 21 days, the tumors were growing to the size of 13 ± 2 mm x 12 ± 3 mm. Then 50 µl of 3 % of sarcosine 1-isobutyl-1H-imidazo[4,5-c]quinolin-4-amide hydrochloride and 1-isobutyl-1H-imidazo[4,5-c]quinolin-4-amide hydrochloride in ethanol/0.2M pH 7.2 phosphate buffer (v/v, 70/30) was topically applied to the human breast cancer cells-implanted area (near the front leg) (twice per day). The tumors are growing to the size of 21 ± 3 mm x 19 ± 3 mm in the control group (n=7) at day 45 and all mice died by the 60^{th} day. In the group (n=7) that was treated with 1-isobutyl-1H-imidazo[4,5-c]quinolin-4-amide hydrochloride, the tumors were 17 ± 2 mm x 15 ± 2 mm at day 45 and all mice died by the day 70. The tumors in the group (n=7) that were treated with sarcosine 1-isobutyl-1H-imidazo[4,5-c]quinolin-4-amide hydrochloride had shrunk to 11 ± 2 mm x 10 ± 2 mm at day 45 and none of mice died at the day 70. The average weights of the mice were 21 ± 2 grams for the sarcosine 1-isobutyl-1H-imidazo[4,5-c]quinolin-4-amide hydrochloride treated group, 22 ± 2 grams for 1-isobutyl-1H-imidazo[4,5-c]quinolin4-amide hydrochloride treated group , and 23 ± 2 grams for the control group at day 45.

The evaluation of anti-herpes activity of these prodrugs was carried out using the method described by Kern, et al. [Antimicrob. Agents Chemother. 14, 817, (1978)]. In the first experiment, female guinea pigs (n=5) were anesthetized. About 100 µl of 3% of sarcosine 1-isobutyl-1H-imidazo[4,5-c]quinolin-4-amide hydrochloride in ethanol/ 0.2M pH 7.4 phosphate buffer (v/v, 70/30) was applied intravaginally to the guinea pigs for 3 days. The guinea pigs were then infected with herpes simplex virus (Type I or Type II, about 10⁵ plaque forming units were used) intravaginally using a cotton swab. Virus replication was monitored by determining the amount of virus recovered with vaginal swabs taken on days 1, 2, 3, 5, or 7 after infection. In the second experiment, the female guinea pigs (n=5) were anesthetized. About 200 µl of 3% of sarcosine 1-isobutyl-1H-imidazo[4,5-c]quinolin-4-amide hydrochloride in ethanol/ 0.2M pH 7.4 phosphate buffer (v/v, 70/30) was applied intravaginally to the guinea pigs and herpes simplex virus (Type I or Type II, about 10⁵ plaque forming units were used) was applied intravaginally to the same guinea pigs using a cotton swab. In the third experiment, the female guinea pigs (n=5) were anesthetized. The guinea pigs were then infected with herpes simplex virus (type I or Type II, about 10⁵ plaque forming units were used) intravaginally using a cotton swab. After 5 days, about 100 µl of 5% of sarcosine 1-isobutyl-1H-imidazo[4,5-c]quinolin-4-amide hydrochloride in ethanol/0.2M pH 7.4 phosphate buffer (v/v, 70/30) was applied intravaginally once per day for 10 days. It has been found that the compound sarcosine 1-isobutyl-1H-imidazo[4,5-c]quinolin-4-amide hydrochloride is effective when administered to guinea pigs beginning before, same time, or after infection.

1H-imidazo[4,5-c]quinolin-4-amines can be prepared using the methods disclosed in U.S. Pat. No. 4, 689, 388. The compounds of the general formula (1) 'Structure 1' or formula (2) 'Structure 2' indicated above can be prepared from 1H-imidazo[4,5-c] quinolin-4-amines or related compounds, by reaction with compounds of the general formula (3) 'Structure 3' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al., 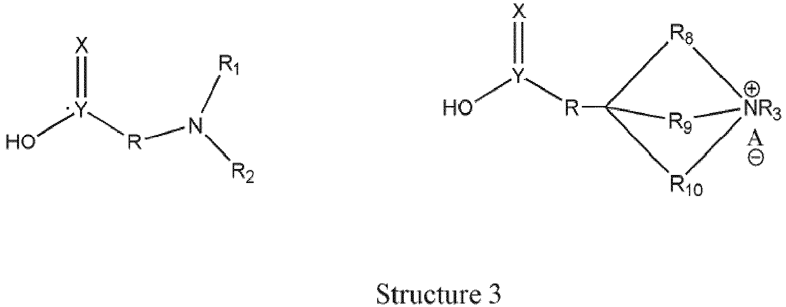 wherein , R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₁₁ R₁₂, aryl or heteroaryl residues, or other ring systems; R₁ and R₂ taken alone are same or different and are H, one of any alkyl, alkyloxyl, alkenyl or alkynyl residues having 1 to 12 carbon atoms, aryl or heteroaryl moieties or taken together are -(CH₂)ₘ-, wherein m=2, 3, 4, 5, 6, 7, 8, 9, 10,..., and any CH₂ may be replaced with O, S, CH=CH, C≡C, CR₁₁R₁₂, aryl or heteroaryl moieties, or other ring moieties; X represents O, NH, or S; Y represents C, S=O, or P-OR₁₁; R₄ represents H, one of any alkyl, alkyloxyl, alkenyl or alkynyl residues having 1 to 12 carbon atoms, aryl or heteroaryl moieties; R₈ represents a branched or straight chain, -(CH₂)ₐ-, wherein a=0, 1, 2, 3, 4, 5, 6, 7, 8, 9, 10 ..., in -(CH₂)ₐ-, any CH₂ may be replaced with O, S, CH=CH, C≡C, CR₁₁R₁₂, aryl or heteroaryl residues, or other ring systems; R₉ represents a branched or straight chain, - (CH₂)_{b}-, wherein b=0, 1, 2, 3, 4, 5, 6, 7, 8, 9, 10 ..., in -(CH₂)_{b}-, any CH₂ may be replaced with O, S, CH=CH, C≡C, CR₁₁R₁₂, aryl or heteroaryl residues, or other ring systems; R₁₀ represents a branched or straight chain, -(CH₂)_{c}-, wherein c=0, 1, 2, 3, 4, 5, 6, 7, 8, 9, 10 ..., in -(CH₂)_{c}-, any CH₂ may be replaced with O, S, CH=CH, C≡C, CR ₁₁R₁₂, aryl or heteroaryl residues, or other ring systems; R₁₁ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁R₂, aryl or heteroaryl moieties, or other ring moieties; R₁₂ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁R₂, aryl or heteroaryl moieties, or other ring moieties; A⁻ represents Cl⁻, Br⁻, F⁻, I⁻, AcO⁻, citrate, or any negative ions. All R, R₁, R₂, R₃, R₄, -(CH₂)ₙ-, -(CH₂)ₘ-, -(CH₂)ₐ-, -(CH₂)_{b}-, or -(CH₂)_{c}- groups are branched or straight chains and may include C, H, O, S, P, or N atoms and may have single, double, and triple bonds.

The compounds of the general formula (1) 'Structure 1' or formula (2) 'Structure 2' indicated above can be prepared from 1H-imidazo[4,5-c]quinolin-4-amines and related compounds, by reaction with compounds of the general formula (4) 'Structure 4', 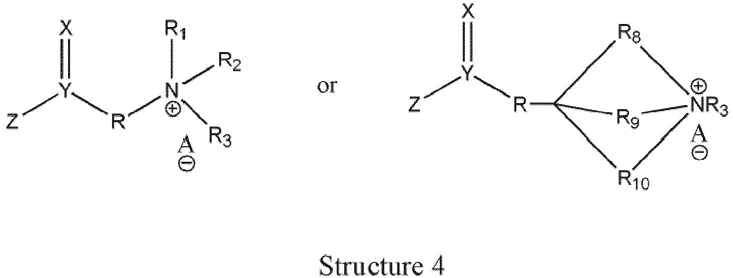 wherein, R represents a branched or straight chain, -(CH₂)ₙ-, wherein n=0, 1, 2, 3, 4, 5, 6, 7, 8, 9, 10 ..., and any CH₂ may be replaced with O, S, CH=CH, C=C, CR₁₁R₁₂, aryl or heteroaryl residues, or other ring systems; R₁ and R₂ taken alone are same or different and are H, one of any alkyl, alkyloxyl, alkynyl, perfluoroalkyl, alkyl halide or alkynyl residues having 1 to 12 carbon atoms, aryl or heteroaryl moieties or taken together are -(CH₂)ₘ-, wherein m=2, 3, 4, 5, 6, 7, 8, 9, 10,..., and any CH may be replaced with O, S, CH=CH, C≡C, CR₁₁R₁₂, aryl or heteroaryl moieties, or other ring moieties; R₃ represent H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁₁R₁₂, aryl or heteroaryl moieties, or other ring moieties; R represents a branched or straight chain,-(CH₂)ₐ-, wherein a=0, 1, 2, 3, 4, 5, 6, 7, 8, 9, 10 ..., in -(CH₂)ₐ-, any CH₂ may be replaced with O, S, CH=CH, C≡C, CR₁₁R₁₂, aryl or heteroaryl residues, or other ring systems; R₉ represents a branched or straight chain, -(CH₂)_{b}-, wherein b=0, 1, 2, 3, 4, 5, 6, 7, 8, 9, 10 ..., in -(CH₂)_{b}-, any CH₂ may be replaced with O, S, CH=CH, C≡C, CR₁₁ R₁₂, aryl or heteroaryl residues, or other ring systems; R₁₀ represents a branched or straight chain, -(CH₂)_{c}-, wherein c=0, 1, 2, 3, 4, 5, 6, 7, 8, 9, 10 ..., in -(CH₂)_{c}-, any CH₂ may be replaced with O, S, CH=CH, C≡C, CR₁₁R₁₂, aryl or heteroaryl residues, or other ring systems; R₁₁ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁ R₂, aryl or heteroaryl moieties, or other ring moieties; R₁₂ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁R₂, aryl or heteroaryl moieties, or other ring moieties; A⁻ represents Cl⁻, Br⁻, F⁻, I⁻, AcO⁻, citrate, or any negative ions. All R, R₁, R₂, R₃, R₄, -(CH₂ )ₙ-, -(CH₂)ₙ-, -(CH₂)ₐ-, -(CH₂)_{b}-, or -(CH₂)_{c}- groups are branched or straight chains and may include C, H, O, S, P, or N atoms and may have single, double, and triple bonds.

### Advantageous Effects

These pro-drugs of 1H-imidazo[4,5-c]quinolin-4-amines and related compounds in the present invention have a lipophilic portion and a hydrophilic portion (the amine groups that exist in the protonated form at physiological pH). The positively charged amino groups of these pro-drugs have two major advantages. First, it largely increases the solubility of the drugs in water; when these new pro-drugs are administered transdermally in a dosage form such as a solution, spray, lotion, ointment, emulsion or gel, they will mix with the moisture on the skin, eye, genital area, mouth, nose, or other part of the body immediately. Second, the positive charge on the amino group of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay on the skin, eye, genital area, mouth, nose, or other part of the body, the pro-drugs will not cause itching, burning, or pain. Experiment results show that more than 90% of the pro-drugs were changed back to the parent drugs in a few minutes. The pro-drugs have a much better absorption rate and as transdermal administration avoids the first pass metabolism, the pro-drugs will be stronger than 1H-imidazo[4,5-c]quinolin-4-amines and related compounds at the same dosage.

### Description of Drawings

Figure 1: Cumulative amounts of sarcosine 1-isobutyl-1H-imidazo[4,5-c] quinolin-4-amide hydrochloride (3% solution, A), sarcosine 1-butyl-1H-imidazo[4,5-c] quinolin-4-amide hydrochloride (3% solution, B), sarcosine 1-benzyl-1H-imidazo[4, 5-c]quinolin-4-amide hydrochloride (3% solution, C), 1-isobutyl-1H-imidazo[4,5-c] quinolin-4-amide hydrochloride (3% suspension, D), 1-butyl-1H-imidazo[4,5-c] quinolin-4-amide hydrochloride (3% suspension, E), and 1-benzyl-1H-imidazo[4,5-c] quinolin-4-amide hydrochloride (3% suspension, F), crossing isolated human skin tissue in Franz cells (n-5). In each case, the vehicle was a mixture of ethanol/pH 7.4 phosphate buffer (0.2 M) (v/v, 70/30).
Figure 2: Wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C=C, CR₁₁R₁₂, aryl or heteroaryl residues, or other ring systems; R₁ and R₂ taken alone are same or different and are H, one of any alkyl, alkyloxyl, alkenyl, perfluoroalkyl, alkyl halide or alkynyl residues having 1 to 12 carbon atoms, aryl or heteroaryl moieties or taken together are -(CH₂)ₘ-, wherein m=2, 3, 4, 5, 6, 7, 8, 9, 10,..., and any CH₂ may be replaced with O, S, CH=CH, C≡C, CR₁₁R₁₂, aryl or heteroaryl moieties, or other ring moieties; R₃ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁₁R₁₂, aryl or heteroaryl moieties, or other ring moieties; X represents O, NH, or S; Y represents C, S=O, or P-OR₁₁; R₄ is selected from the group consisting of alkyl of 1 to about 10 carbon atoms, hydroxylalkyl of 1 to about 10 carbon atoms, acyloxyalkyl wherein the acyloxy moiety is alkanoyloxy of 2 to about 5 carbon atoms or benzoyloxy, and the alkyl moiety contains 1 to about 8 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to about 5 carbon atoms, alkoxy of 1 to about 5 carbon atoms and halogen, with the proviso that if said benzene ring is substituted by 2 of said moieties, then said moieties together contain no more than 8 carbon atoms; R₅ is selected from the group consisting of hydrogen, alkyl of 1 to about 10 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to about 5 carbon atoms, alkoxy of 1 to about 5 carbon atoms and halogen, with the proviso that if said benzene ring is substituted by 2 of said moieties, then said moieties together contain no more than 8 carbon atoms; R₆ is selected from the group consisting of hydrogen, alkyl of 1 to about 5 carbon atoms, and alkoxy of 1 to about 5 carbon atoms and halogen; R₇ is selected from the group consisting of hydrogen, alkyl of 1 to about 5 carbon atoms, and alkoxy of 1 to about 5 carbon atoms and halogen; R₈ represents a branched or straight chain,-(CH₂)ₐ-, wherein a=0, 1, 2, 3, 4, 5, 6, 7, 8, 9, 10 ..., in -(CH₂)ₐ-, any CH₂ may be replaced with O, S, CH=CH, C≡C, CR₁₁R₁₂, aryl or heteroaryl residues, or other ring systems; R₉ represents a branched or straight chain, -(CH₂)_{b}-, wherein b=0, 1, 2, 3, 4, 5, 6, 7, 8, 9, 10 ..., in -(CH₂)_{b}-, any CH may be replaced with O, S, CH=CH, C≡C, CR₁₁ R₁₂, aryl or heteroaryl residues, or other ring systems; R₁₀ represents a branched or straight chain, -(CH₂)_{c}-, wherein c=0, 1, 2, 3, 4, 5, 6, 7, 8, 9, 10 ..., in -(CH₂)_{c-}, any CH₂ may be replaced with O, S, CH=CH, C≡C. CR₁₁R₁₂, aryl or heteroaryl residues, or other ring systems; R₁₁ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁ R₂, aryl or heteroaryl moieties, or other ring moieties; R₁₂ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁R₂, aryl or heteroaryl moieties, or other ring moieties; A⁻ represents Cl⁻, Br⁻, F, I⁻, AcO⁻, citrate, or any negative ions. All R, R₁, R₂, R₃, R₄, -(CH₂ )ₙ-, -(CH₂)ₘ-, -(CH₂)ₐ-, -(CH₂)_{b}-, or -(CH₂)_{c}- groups are branched or straight chains and may include C, H, S, P, or N atoms and may have single, double, and triple bonds.

### Best Mode

### Preparation of Boc-sarcosine 1-isobutyl-1H-imidazo[4,5-c]quinolin-4-aimde

32.2 g (0.1 mol) of 1-isobutyl-1H-imidazo[4,5-c]quinolin-4-amine was dissolved in 500 ml of ethyl acetate. 36 g (0.1 mol) of N-t-Butyloxycarbonyl-N-methylglycine anhydride [(Boc-N-Me-Gly)₂O] and 20 ml of triethylamine were added into the reaction mixture. The mixture was refluxed for 2 h. The solution was washed with water (1 x 100 ml), 10% citric acid (1 x 100 ml), water (1 x 100 ml), 5% sodium bicarbonate (1 x 100 ml), and water (3 x 100 ml). The ethyl acetate solution is dried over anhydrous sodium sulfate. The ethyl acetate solution was evaporated to dryness. After drying, it yielded 36 g of the desired product (87.5%). Elementary analysis: C₂₂H₂₉N₅O_{3;} MW: 411.50. Calculated % C: 64.21; H: 7.10; N: 17.02; O: 11.66; Found % C: 64.17; H: 7.15; N: 16.97; O: 11.71. ¹H-NMR (400 MHz, CDCl₃): δ: 1.12 (d, 6H), 1.52 (d, 9H), 2,37 (m, H), 3.05 (s, 3H), 4.30 (d, 2H), 4.78 (m, 2H), 7.60-7.70 (m, 2H), 7.85 (s, H), 8.02-8.20 (m, 2H), 9.01 (b, H).

### Preparation of sarcosine 1-isobutyl-1H-imidazo[4,5-c]quinolin-4-amide hydrochloride

35 g of Boc-sarcosine 1-isobutyl-1H-imidazo[4,5-c]quinolin-4-amide was dissolved in ethanol (200 ml). HCl gas was bubbled into the reaction mixture for 20 min. slowly. The mixture was stirred for 1 hour at RT. Ether (500 ml) was added into the mixture. The solid was collected by filtration and washed with ether (3 x). After drying, it yielded 27 g of the desired product (91.2%). Elementary analysis: C₁₇H₂₂ClN₅ O; MW: 347.84. Calculated % C: 58.70; H: 6.37; Cl: 10.19; N: 20.13; O: 4.60; Found % C: 58.67; H: 6.41; Cl: 10.17; N: 20.11; O: 4.64.

### Mode for Invention

### Preparation of Boc-Glycine 1-butyl-1H-imidazo[4,5-c]quinolin-4-amide

32.2 g (0.1 mol) of 1-butyl-1H-imidazo[4,5-c]quinolin-4-amme was dissolved in 300 ml of ethyl acetate. 33.3 g (0.1 mol) of N-t-Butyloxycarbonyl-glycine anhydride [(Boc-Gly)₂O] and 20 ml of triethylamine were added into the reaction mixture. The mixture was refluxed for 2 h. The solution was washed with water (1 x 100 ml), 10% citric acid (1 x 100 ml), water (1 x 100 ml), 5% sodium bicarbonate (1 x 100 ml), and water (3 x 100 ml). The ethyl acetate solution is dried over anhydrous sodium sulfate. The ethyl acetate solution was evaporated to dryness. After drying, it yielded 37 g of the desired product (93.1%). Elementary analysis: C₂₁H₂₇N₅O_{3;} MW: 397.47. Calculated % C: 63.46; H: 6.85; N: 17.62; O: 12.08; Found % C: 63.42; H: 6.87; N: 17.60; O: 12.11. ¹H-NMR (400 MHz, CDCl₃): δ: 0.93 (t, 3H), 1.27 (m, 2H), 1.45 (d, 9H), 1.68 (m, 2H), 4.30 (m, 2H), 4.76 (m, 2H), 7.60-7.71 (m, 2H), 7.85 (s, H), 8.02-8.17 (m, 2H), 8.62 (b, H), 9.02 (b, H).

### Preparation of glycine 1-isobutyl]-1H-imidazo[4,5-c]quinolin-4-amide hydrochloride

36 g of Boc-glycine 1-isobutyl-1H-imidazo[4,5-c]quinolin-4-amide was dissolved in ethanol (200 ml). HCl gas was bubbled into the reaction mixture for 20 min. slowly. The mixture was stirred for 1 hour at RT. Ether (500 ml) was added into the mixture. The solid was collected by filtration and washed with ether (3 x). After drying, it yielded 28 g of the desired product (92.6%). Elementary analysis: C₁₆H₂₀ClN₅O; MW: 333.82. Calculated % C: 57.57; H: 6.04; Cl: 10.62; N: 20.98; O: 4.79; Found % C: 57.52; H: 6.08; Cl: 10.67; N: 20.95; O: 4.78.

### Preparation of N,N-dimethylglycine 1-butyl-1H-imidazo[4,5-c] quinolin-4-amide hydrochloride

Glycine 1-butyl-1H-imidazo[4,5-c]quinolin-4-amide hydrochloride (27 g) was dissolved in 2N NaOH (50 ml). 50 ml of 40% formaldehyde and 50 ml of acetic acid were added into the reaction mixture. 30 g of NaBH₄ was added into the reaction mixture slowly. After addition, the mixture is stirred for 30 min. Another 25 ml of 40% formaldehyde and 10 ml of acetic acid was added into the reaction mixture. 20 g of NaBH₄ was added into the reaction mixture slowly. The mixture is evaporated to dryness. The residue was purified by silica gel column chromatography. Yielded 20 g of desired product (68.8%). Elementary analysis: C₁₈H₂₄ClN₅O; MW: 361.87. Calculated % C: 59.74; H: 6.68; Cl: 9.80; N: 19.35; O: 4.42; Found % C: 59.72; H: 6.72; Cl: 9.75; N: 19.37; O: 4.44.

### Preparation of Boc-sarcosine 1-benzyl-1H-imidazo[4,5-c]quinolin-4-amide

18.9 g (0.1 mol) of N-t-Butyloxycarbonyl-N-methylglycine was dissolved in 300 ml of dichloromethlene. 20.6 g of N, N'-Dicyclohexylcarbodiimide was added into the reaction mixture. The mixture was stirred for 1 hour at 0°C. 32.2 g (0.1 mol) of 1-benzyl-1H-imidazo[4,5-c]quinolin-4-amine and 20 ml of triethylamine were added into the reaction mixture. The mixture was stirred for 3 hours at RT. The solid was removed by filtration. The dichloromethlene solution was washed with water (1 x 100 ml), 30% citric acid (1 x 100 ml), water (1 x 100 ml), 5% NaHCO₃ (2 x 100 ml), and water (3 x 100 ml). The organic solution was dried over anhydrous sodium sulfate. Sodium sulfate was removed by filtration. The organic solution was evaporated to dryness. After drying, it yielded 33 g of the desired product (74.1%). Elementary analysis: C₂₅H₂₇N₅O₃; MW: 445.51. Calculated % C: 67.40; H: 6.1.1; N: 15.72; O: 10.77; Found % C: 67.35; H: 6.14; N: 15.70; O: 10.81. ¹H-NMR (400 MHz, CDCl₃): δ : 1.52 (d, 9H), 3.02 (s, 3H), 4.68 (m, 2H), 4.98 (m, 2H), 7.10-7.16 (m, 5H), 7.60-7.70 (m, 2H), 7.85 (s, H), 8.02-8.20 (m, 2H), 8.91 (b, H).

### Preparation of sarcosine 1-benzyl-1H-imidazo[4,5-c]quinolin-4-amide hydrochloride

32 g of Boc-sarcosine 1-benzyl-1H-imidazo[4,5-c]quinolin-4-amide was dissolved in ethanol (200 ml). HCl gas was bubbled into the reaction mixture for 20 min. slowly. The mixture was stirred for 1 hour at RT. Ether (500 ml) was added into the mixture. The solid was collected by filtration and washed with ether (3 x). After drying, it yielded 25 g of the desired product (85.7%). Elementary analysis: C₂₀H₂₀ClN₅O; MW: 381.86. Calculated % C: 62.91; H: 5.28; Cl: 9.28; N: 18.34; O: 4.19; Found % C: 62.87; H: 5.31; Cl: 9.30; N: 18.32; O: 4.20.

### Industrial Applicability

The pro-drugs of the general formula (1) 'Structure 1' or formula (2) 'Structure 2' are superior to 1H-imidazo[4,5-c]quinolin-4-amines and related compounds. They can be used medicinally in treating any 1H-imidazo[4,5-c]quinolin-4-amines and related compounds-treatable conditions in humans or animals. They may be used for the treatment of actinic keratosis, basal cell carcinoma, cancers, AIDs, bird flu, genital and perianal warts, and other virus diseases.

The present invention therefore provides:
[1] A compound corresponding to the general formula (1) "Structure 1" or formula (2) "Structure 2", 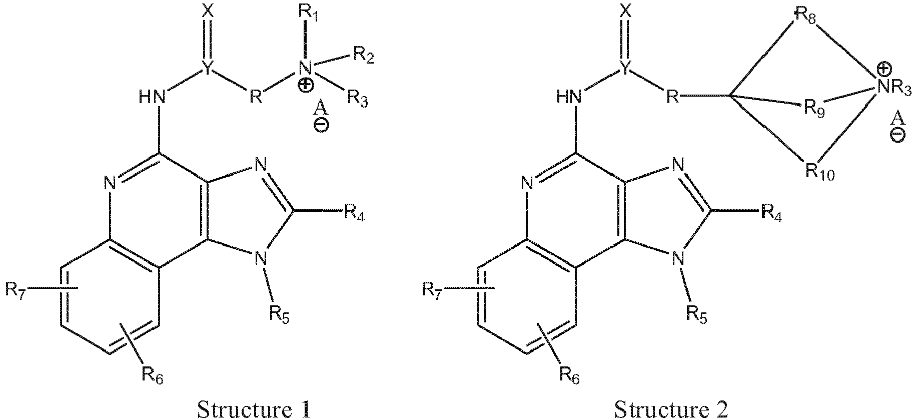 wherein, R represents a branched or straight chain, -(CH₂)ₙ -,wherein n=O, 1, 2, 3, 4, 5, 6, 7, 8, 9, 10 ... ,in -(CH₂)ₙ -,any CH₂ may be replaced with O, S, CH=CH, C≡C, CR₁₁R₁₂, aryl or heteroaryl esidues, or other ring systems; R₁ and R₂ taken alone are same or different and are H, one of any alkyl, alkyloxyl, alkenyl, perfluoroalkyl, alkyl halide or alkynyl residues having 1 to 15 carbon atoms, aryl or heteroaryl moieties or taken together are -(CH₂)ₘ -, wherein m=2, 3, 4, 5, 6, 7, 8, 9, 10,... , and any CH₂ may be replaced with 0, S, CH=CH, C:::C, CR₁₁R₁₂, aryl or heteroaryl moieties, or other ring moieties; R₃ represents H, one of any alkyl, alkyloxy, alkenyl, perfluoroalkyl, alkyl halide, or alkynyl residues having 1 to 15 carbon atoms, aryl or heteroaryl moieties, wherein, any CH₂ may be replaced with O, S, CH=CH, C≡C, CR₁₁R₁₂, aryl or heteroaryl moieties, or other ring moieties; X represents O, NH, or S; Y represents C, S=O, or P-OR₁₁; R₄ is selected from the group consisting of alkyl of 1 to about 10 carbon atoms, hydroxylalkyl of 1 to about 10 carbon atoms, acyloxyalkyl wherein the acyloxy moiety is alkanoyloxy of 2 to about 5 carbon atoms or benzoyloxy, and the alkyl moiety contains 1 to about 8 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to about 5 carbon atoms, alkoxy of 1 to about 5 carbon atoms and halogen, with the proviso that if said benzene ring is substituted by 2 of said moieties, then said moieties together contain no more than 8 carbon atoms; R₅ is selected from the group consisting of hydrogen, alkyl of 1 to about 10 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to about 5 carbon atoms, alkoxy of 1 to about 5 carbon atoms and halogen, with the proviso that if said benzene ring is substituted by 2 of said moieties, then said moieties together contain no more than 8 carbon atoms; R₆ is selected from the group consisting of hydrogen, alkyl of 1 to about 5 carbon atoms, and alkoxy of 1 to about 5 carbon atoms and halogen; R₇ is selected from the group consisting of hydrogen, alkyl of 1 to about 5 carbon atoms, and alkoxy of 1 to about 5 carbon atoms and halogen; R₈ represents a branched or straight chain, -(CH₂)ₐ -, wherein a=0, 1, 2, 3, 4, 5, 6, 7, 8, 9, 10 ... ,in -(CH₂)ₐ, any CH₂ may be replaced with O, S, CH=CH, C≡C, CR₁₁R₁₂, aryl or heteroaryl residues, or other ring systems; R₉ represents a branched or straight chain, -(CH₂)_{b} -,wherein b=0, 1, 2, 3, 4, 5, 6, 7, 8, 9, 10 ... , in -(CH₂)_{b} -,any CH₂ may be replaced with 0, S, CH=CH, C≡C, CR₁₁R₁₂, aryl or heteroaryl residues, or other ring systems; R10 represents a branched or straight chain, -(CH₂)_{C}-,wherein c=0, 1, 2, 3, 4, 5, 6, 7, 8, 9, 10 ... , in-(CH₂)_{c} -, any CH₂ may be replaced with O, S, CH=CH, C≡C, CR₁₁ R₁₂, aryl or heteroaryl residues, or other ring systems; R₁₁ represents H, one of any alkyl, alkyloxy, alkenyl, perfluoroalkyl, alkyl halide, or alkynyl residues having 1 to 15 carbon atoms, aryl or heteroaryl moieties, wherein, any CH₂ may be replaced with O, S, CH=CH, C≡C, CR R , aryl or heteroaryl moieties, or other ring moieties; R₁₂ represents H, one of any alkyl, alkyloxy, alkenyl, perfluoroalkyl, alkyl halide, or alkynyl residues having 1 to 15 carbon atoms, aryl or heteroaryl moieties, wherein, any CH₂ may be replaced with O, S, CH=CH, C=C, CR₁R₂, aryl or heteroaryl moieties, or other ring moieties; A represents Cl⁻, Br⁻, F⁻, I⁻, AcO⁻, citrate, or any negative ions. All R, R₁, R₂, R₃, R₄, -(CH₂)ₙ -, -(CH₂)ₘ -, -(CH₂)ₐ -, -(CH₂)_{b} -, or -(CH₂)_{c} - groups are branched or straight chains and may include C, H, O, S, P, or N atoms and may have single, double, and triple bonds.
[2] A compound as in [1], which is sarcosine 1-isobutyl-1H-imidazo[4,5-c]quinolin-4-amide.HA, wherein, A⁻ represents Cl⁻, Br⁻, F⁻, I⁻, AcO⁻, citrate, or any negative ions.
[3] A compound as in [1], which is N,N-dimethylglycine 1-isobutyl-1H-imidazo[4, 5-c]quinolin-4-amide.HA, wherein, A⁻ represents Cl⁻, Br⁻, F⁻, I⁻, AcO⁻, citrate, or any negative ions.
[4] A compound as in [1], which is sarcosine 1-butyl-1H-imidazo[4,5-c]quinolin-4-amide.HA, wherein, A⁻ represents Cl⁻, Br⁻, F⁻, I⁻, AcO⁻, citrate, or any negative ions.
[5] A compound as in [1], which is sarcosine 1-benzyl-1H-imidazo[4,5-c]quinolin-4-amide.HA, wherein, A⁻ represents Cl⁻, Br⁻, F⁻, I⁻, AcO⁻, citrate, or any negative ions.
[6] A compound as in [1], which is sarcosine 1-methyl-1H-imidazo[4,5-c]quinolin-4-amide.HA, wherein, A⁻ represents Cl⁻, Br⁻, F⁻, I⁻, AcO⁻, citrate, or any negative ions.
[7] A compound as in [1], which is sarcosine 1, 2, 8-trimethyl-1H-imidazo[4,5-c]quinolin-4-amide.HA, wherein, A⁻ represents Cl⁻, Br⁻, F⁻, I⁻, AcO⁻, citrate, or any negative ions.
[8] A compound as in [1], which is sarcosine 1-(2-hydroxyethyl)-1H-imidazo[4,5-c]quinolin-4-amide.HA, wherein, A⁻ represents Cl⁻, Br⁻, F⁻, I⁻, AcO⁻, citrate, or any negative ions.
[9] A compound as in [1], which is sarcosine 1,8-dimethyl-1H-imidazo[4,5-c]quinolin-4-amide.HA, wherein, A⁻ represents Cl⁻, Br⁻, F⁻, I⁻, AcO⁻, citrate, or any negative ions.
[10] A compound as in [1], which is sarcosine 1,2-dimethyl-1H-imidazo[4,5-c]quinolin-4-amide.HA, wherein, A⁻ represents Cl⁻, Br⁻, F⁻, I⁻, AcO⁻, citrate, or any negative ions.
[11] A compound as in [1], which is sarcosine 1-(2,3-dihydroxypropyl)-1H-imidazo[4,5-c]quinolin-4-amide.HA, wherein, A⁻ represents Cl⁻, Br⁻, F⁻, I⁻, AcO⁻, citrate, or any negative ions.
[12] A compound as in [1], which is sarcosine 1-cyclohexylmethyl-1H-imidazo[4,5-c]quinolin-4-amide.HA, wherein, A⁻ represents Cl⁻, Br⁻, F⁻, I⁻, AcO⁻, citrate, or any negative ions.
[13] A compound as in [1], which is sarcosine 1-benzyl-2-methyl-1H-imidazo[4,5-c]quinolin-4-amide.HA, wherein, A⁻ represents Cl⁻, Br⁻, F⁻, I⁻, AcO⁻, citrate, or any negative ions.
[14] A compound as in [1], which is sarcosine 1-n-hexyl-1H-imidazo[4,5-c]quinolin-4-amide.HA, wherein, A⁻ represents Cl⁻, Br⁻, F⁻, I⁻, AcO⁻, citrate, or any negative ions.
[15] A compound as in [1], which is sarcosine 1-n-hexyl-2-methyl-1H-imidazo[4,5-c]quinolin-4-amide.HA, wherein, A⁻ represents Cl⁻, Br⁻, F⁻, I⁻, AcO⁻, citrate, or any negative ions.
[16] A design of prodrugs should have a lipophilic portion and a primary, secondary, or tertiary amine group that exists in the protonated form (hydrophilic portion) at physiological pH, and should have only one or two (preferably one) primary, secondary, or tertiary amine groups that exists in the protonated form (hydrophilic portion) at physiological pH. any carboxyl groups, amino groups, guanidine groups or other hydrophilic groups in the drugs can be protected with an alkyl, aryl, or heteroaryl ester or amide group to make the prodrugs lipophilic.
[17] Processes for the preparation of compounds of the general formula (1) "Structure 1" or formula (2) "Structure 2" according to [1] to [15], wherein the compounds can be prepared from 1H-imidazo[4,5-c]quinolin-4-amines or related compounds, by reaction with compounds of the general formula (3) "Structure 3" by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al. 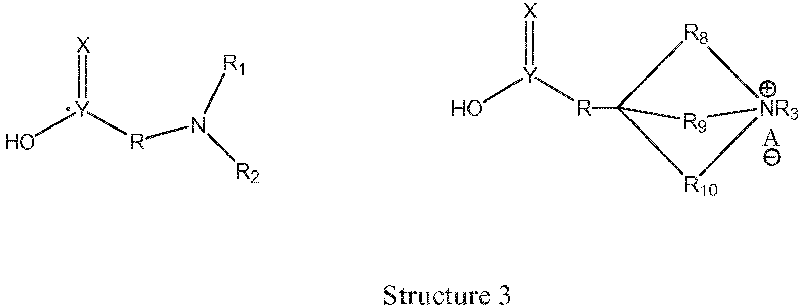 wherein, R represents a branched or straight chain, -(CH₂)ₙ-, wherein n=0, 1, 2, 3, 4, 5, 6, 7, 8, 9, 10 ..., in -(CH₂)ₙ-, any CH₂ may be replaced with O, S, CH=CH, C≡C, CR₁₁R₁₂, aryl or heteroaryl residues, or other ring systems; R₁ and R₂ taken alone are same or different and are H, one of any alkyl, alkyloxyl, alkenyl or alkynyl residues having 1 to 15 carbon atoms, aryl or heteroaryl moieties or taken together are -(CH₂)ₘ-, wherein m=2, 3, 4, 5, 6, 7, 8, 9, 10,..., and any CH₂ may be replaced with O, S, CH=CH, C≡C, CR₁₁R₁₂, aryl or heteroaryl moieties, or other ring moieties; X represents O, NH, or S; Y represents C, S=O, or P-OR₁₁; R₄ represents H, one of any alkyl, alkyloxyl, alkenyl, or alkynyl residues having 1 to 15 carbon atoms, aryl or heteroaryl moieties; R₈ represents a branched or straight chain, -(CH₂)ₐ-, wherein a=0, 1, 2, 3, 4, 5, 6, 7, 8, 9, 10 ..., in -(CH₂)ₐ-, any CH₂ may be replaced with O, S, CH=CH, C≡C, CR₁R₂, aryl or heteroaryl residues, or other ring systems; R₉ represents a branched or straight chain,-(CH₂)_{b}-, wherein b=0, 1, 2, 3, 4, 5, 6, 7, 8, 9, 10 ..., in -(CH₂)_{b}-, any CH₂ may be replaced with O, S, CH=CH, C≡C, CR₁₁R₁₂, aryl or heteroaryl residues, or other ring systems; R₁₀ represents a branched or straight chain, -(CH₂)_{c}-, wherein c=0, 1, 2, 3, 4, 5, 6, 7, 8, 9, 10 ..., in -(CH₂)_{c}-, any CH₂ may be replaced with O, S, CH=CH, C≡C, CR₁₁R₁₂, aryl or heteroaryl residues, or other ring systems; R₁₁ represents H, one of any alkyl, alkyloxy, alkenyl, perfluoroalkyl, alkyl halide, or alkynyl residues having 1 to 15 carbon atoms, aryl or heteroaryl moieties, wherein, any CH may be replaced with O, S, CH=CH, C≡C, CR₁₁R₁₂, aryl or heteroaryl moieties, or other ring moieties; R₁₂ represents H, one of any alkyl, alkyloxy, alkenyl, perfluoroalkyl, alkyl halide, or alkynyl residues having 1 to 15 carbon atoms, aryl or heteroaryl moieties, wherein, any CH₂ may be replaced with O, S, CH=CH, C≡C, CR₁R₂, aryl or heteroaryl moieties, or other ring moieties; A⁻ represents Cl⁻, Br⁻, F⁻, I⁻, AcO⁻, citrate, or any negative ions. All R, R₁, R₂, R₃, R₄, -(CH₂)ₙ-,-(CH₂)ₘ-, -(CH₂)ₐ-, -(CH₂)_{b}-, or -(CH₂)_{c}- groups are branched or straight chains and may include C, H, O, S, P, or N atoms and may have single, double, and triple bonds.
[18] Processes for the preparation of the compounds of the general formula (1) 'Structure 1' or formula (2) 'Structure 2' according to [1] to [15], wherein the compounds can be prepared from 1H-imidazo[4,5-c]quinolin-4-amines and related compounds, by reaction with compounds of the general formula (4) 'Structure 4', 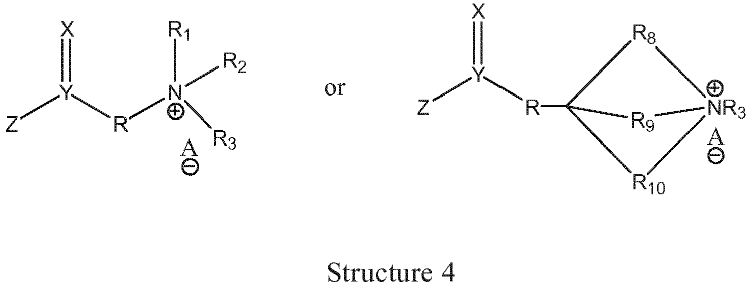 wherein, R represents a branched or straight chain, -(CH₂)ₙ-, wherein n=0, 1, 2, 3, 4, 5, 6, 7, 8, 9, 10 ..., and any CH₂ may be replaced with O, S, CH=CH, C≡C, CR₁₁R₁₂, aryl or heteroaryl residues, or other ring systems; R₁ and R₂ taken alone are same or different and are H, one of any alkyl, alkyloxyl, alkenyl, perfluoroalkyl, alkyl halide or alkynyl residues having 1 to 15 carbon atoms, aryl or heteroaryl moieties or taken together are -(CH₂)ₘ-, wherein m=2, 3, 4, 5, 6, 7, 8, 9, 10,..., and any CH₂ may be replaced with O, S, CH=CH, C≡C, CR₁₁R₁₂, aryl or heteroaryl moieties, or other ring moieties; R₃ represents H, one of any alkyl, alkyloxy, alkenyl, perfluoroalkyl, alkyl halide, or alkynyl residues having 1 to 15 carbon atoms, aryl or heteroaryl moieties, wherein, any CH₂ may be replaced with O, S, CH=CH, C≡C, CR₁₁R₁₂, aryl or heteroaryl moieties, or other ring moieties; R₈ represents a branched or straight chain, -(CH₂)ₐ-, wherein a=0, 1, 2, 3, 4, 5, 6, 7, 8, 9, 10 ..., in -(CH₂)ₐ-, any CH₂ may be replaced with O, S, CH=CH, C≡C, CR₁₁R₁₂, aryl or heteroaryl residues, or other ring systems; R₉ represents a branched or straight chain, -(CH₂)_{b}-, wherein b=0, 1, 2, 3, 4, 5, 6, 7, 8, 9, 10 ..., in -(CH₂)_{b}-, any CH₂ may be replaced with O, S, CH=CH, C≡C, CR₁₁R₁₂, aryl or heteroaryl residues, or other ring systems; R₁₀ represents a branched or straight chain, -(CH₂)_{c}-, wherein c=0, 1, 2, 3, 4, 5, 6, 7, 8, 9, 10 ..., in -(CH₂)_{c}-, any CH₂ may be replaced with O, S, CH=CH, C≡C, CR₁₁R₁₂, aryl or heteroaryl residues, or other ring systems; R₁₁ represents H, one of any alkyl, alkyloxy, alkenyl, perfluoroalkyl, alkyl halide, or alkynyl residues having 1 to 15 carbon atoms, aryl or heteroaryl moieties, wherein, any CH₂ may be replaced with O, S,CH=CH, C≡C, CR₁R₂, aryl or heteroaryl moieties, or other ring moieties; R₁₂ represents H, one of any alkyl, alkyloxy, alkenyl, perfluoroalkyl, alkyl halide, or alkynyl residues having 1 to 15 carbon atoms, aryl or heteroaryl moieties, wherein, any CH₂ may be replaced with O, S, CH=CH, C≡C, CR₁R₂, aryl or heteroaryl moieties, or other ring moieties; A⁻ represents Cl⁻, Br⁻, F⁻, I⁻, AcO⁻, citrate, or any negative ions. All R, R₁, R₂, R₃, R₄, -(CH₂)ₙ-, -(CH₂)ₘ-, -(CH₂)ₐ-, -(CH₂)_{b}-, or -(CH₂)_{c}- groups are branched or straight chains and may include C, H, O, S, P, or N atoms and may have single, double, and triple bonds.
[19] A compound of the general formulas (1) "Structure 1" or formula (2) "Structure 2" or a composition comprising of at least one compound of the general formulas (1) " Structure 1" or formula (2) "Structure 2", as an active ingredient, according to [1] to [15], can be administered orally or transdermally, for treating any 1H-imidazo[4,5-c]quinolin-4-amines or related compounds-treatable conditions in humans or animals. The 1H-imidazo[4,5-c]quinolin-4-amines or related compounds-treatable conditions include, but are not limited to acquired immune deficiency syndrome (AIDS), bird flu, Hydrophobia (rabies infection), warts, severe acute respiratory syndrome (SARS), eczema, hepatitis, psoriasis, multiple sclerosis, essential thrombocythaemia, and other virus diseases, actinic keratosis, basal cell carcinoma, skin cancers, breast cancer, colon cancer, lung cancer, oral cancers and other various cancers.
[20] A method for treating 1H-imidazo[4,5-c]quinolin-4-amines or related compounds-treatable conditions in humans or animals by administering transdermally to any part of body (in the form of a solution, spray, lotion, ointment, emulsion or gel) to deliver therapeutically effective plasma levels of the compounds of the general formulas (1) " Structure 1" or or formula (2) "Structure 2", as an active ingredient, according to [1] to [15].
[21] A method for topically treating warts, severe acute respiratory syndrome (SARS), acquired immune deficiency syndrome (AIDS), bird flu, hydrophobia (rabies infection), and other virus diseases, actinic keratosis, basal cell carcinoma, skin cancers, breast cancer, colon cancer, lung cancer, oral cancers and other various cancers in humans or animals by administering transdermally to the particular site of disease a therapeutically effective amount of the compounds of the general formulas (1) " Structure 1" or formula (2) "Structure 2", as an active ingredient, according to [1] to [15].
[22] A method for the treating or the prevention of acquired immune deficiency syndrome (AIDS) by administering transdermally (in the form of a solution, spray, lotion, ointment, emulsion or gel) to the upper respiratory tract or/and the genital area of a male or female human therapeutically effective levels of the compounds of the general formulas (1) " Structure 1" or formula (2) "Structure 2", as an active ingredient, according to [1] to [15].
[23] A method for the prevention of acquired immune deficiency syndrome (AIDS) by mixing condom materials or coating (in the form of a solution, spray, lotion, ointment, emulsion or gel) on the condom with therapeutically effective levels of the compounds of the general formula (1) " Structure 1" or formula (2) "Structure 2", as an active ingredient, according to [1] to [15].
[24] Transdermal therapeutic application systems of compounds of general formula (1) " Structure 1" or formula (2) "Structure 2", as an active ingredient, according to [1] to [15], for treating any 1H-imidazo[4,5-c]quinolin-4-amines and related compounds-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables the 1H-imidazo[4,5-c]quinolin-4-amines and related compounds to reach constantly optimal therapeutic blood levels to increase effectiveness and reduce the side effects of 1H-imidazo[4,5-c]quinolin-4-amines and related compounds.
[25] A method for treating cancers by administering orally or transdermally the compound of the general formula (1) " Structure 1" or formula (2) "Structure 2", or a composition comprising of at least one compound of the general formulas (1) 'Structure 1' or formula (2) 'Structure 2' , as an active ingredient, according to [1] to [15], wherein, these new prodrugs can be combined with any nonsteroidal anti-inflammatory drugs or their prodrugs, such as diethylaminoethyl 2-(p-isobutylphenyl) propionate.AcOH, diethylaminoethyl 1-(p-chlorobenzoyl)-5-methoxy-2-methylindole 3-acetate.AcOH, diethylaminoethyl (Z)-5-fluoro-2-methyl-1-[(4-methylsulfinyl) phenylmethylene] -1H -indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, diethylaminoethyl 1,8-diethyl-1,3,4,9-tetrahydropyrano-[3,4-b]indole-1-acetate.AcOH, or other pharmaceuticals.
[26] A method for treating cancers by administering orally or transdermally a compound of the general formula (1) 'Structure 1' or formula (2) 'Structure 2' or a composition comprising of at least one compound of the general formulas (1) 'Structure 1' or formula (2) 'Structure 2', as an active ingredient, according to [1] to [15] before or/and after removing tumor surgery.

## Claims

1. A compound of formula (1) or formula (2), 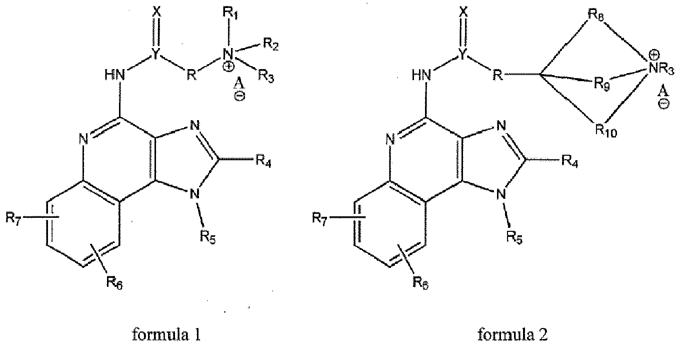 wherein,
R is a branched or straight chain -(CH₂)ₙ-, wherein n=1, 2, 3, 4, 5, 6, 7, 8, 9, or 10 and any CH₂ may be replaced with O, S, CH=CH, or C≡C;
R₁ and R₂ are independently selected from H, C₁₋₁₅ alkyl, C₁₋₁₅ alkyloxyl, C₂₋₁₅ alkenyl, C₁₋₁₅perfluoroalkyl, C₁₋₁₅ alkyl halide, and C₂₋₁₅ alkynyl;
R₃ is H, C₁₋₁₅ alkyl, C₁₋₁₅ alkyloxy, C₂₋₁₅ alkenyl, C₁₋₁₅perfluoroalkyl, C₁₋₁₅ alkyl halide, or C₂₋₁₅ alkynyl;
X is O, NH, or S;
Y is C, S=O, or P-OR₁₁;
R₄ is selected from H, C₁₋₁₀ alkyl, C₁₋₁₀ hydroxylalkyl, and acyloxyalkyl, wherein the acyloxy moiety is C₂₋₅ alkanoyloxy or benzoyloxy and the alkyl moiety contains 1 to 8 carbon atoms, benzyl, (phenyl)ethyl and phenyl, wherein the benzyl, (phenyl)ethyl, or phenyl substituent is unsubstituted or substituted on the benzene ring by 1 or 2 moieties independently selected from C₁₋₁₅ alkyl, C₁₋₅ alkoxy, and halogen, with the proviso that if said benzene ring is substituted by 2 of said moieties, then said moieties together contain no more than 8 carbon atoms;
R₅ is selected from H, C₁₋₁₀ alkyl being unsubstituted or substituted by 1 or 2 moieties independently selected from hydroxy or cyclohexyl, benzyl, (phenyl)ethyl, and phenyl, said benzyl, (phenyl)ethyl, or phenyl substituent being unsubstituted or substituted on the benzene ring by 1 or 2 moieties independently selected from C₁₋₅ alkyl, C₁₋₅ alkoxy, and halogen, with the proviso that if said benzene ring is substituted by 2 of said moieties, then said moieties together contain no more than 8 carbon atoms;
R₆ is selected from H, C₁₋₅ alkyl, C₁₋₅ alkoxy, and halogen;
R₇ is selected from H, C₁₋₅ alkyl, C₁₋₅ alkoxy, and halogen;
R₈ is a branched or straight chain, -(CH₂)ₐ-, wherein a=1, 2, 3, 4, 5, 6, 7, 8, 9, or 10 and any CH₂ may be replaced with O, S, CH=CH, or C≡C;
R₉ is absent or a branched or straight chain, -(CH₂)_{b}-, wherein b= 1, 2, 3, 4, 5, 6, 7, 8, 9, or 10 and any CH₂ may be replaced with O, S, CH=CH, or C≡C;
R₁₀ is a branched or straight chain, -(CH₂)_{c}-, wherein c=1, 2, 3, 4, 5, 6, 7, 8, 9, or 10 and any CH₂ may be replaced with O, S, CH=CH, or C≡C;
R₁₁ is H, C₁₋₁₅ alkyl, C₁₋₁₅ alkyloxy, C₂₋₁₅ alkenyl, C₁₋₁₅ perfluoroalkyl, C₁₋₁₅ alkyl halide, or C₂₋₁₅ alkynyl; and
A⁻ is a pharmaceutically acceptable negative ion.

2. The compound of claim 1, wherein the compound is a compound of formula (1).

3. The compound of claim 1, wherein the compound is a compound of formula (2).

4. The compound of any one of claims 1 to 3, wherein R₁ and R₂ are independently C₁₋₁₅ alkyl.

5. The compound of any one of claims 1 to 4, wherein R₄ is H or C₁₋₁₀ alkyl.

6. The compound of any one of claims 1 to 5, wherein A⁻ is Cl⁻, Br⁻, F⁻, I⁻, AcO⁻, or citrate.

7. The compound of any one of claims 1 to 6, wherein R₁ and R₂ are both H.

8. The compound of any one of claims 1 to 6, wherein R₃ is H.

9. A process for the preparation of a compound of the formula (1) or formula (2) according to any one of claims 1 to 8, wherein the compound is prepared from 1H-imidazo[4,5-c]quinolin-4-amine or a related compound of chemical structure 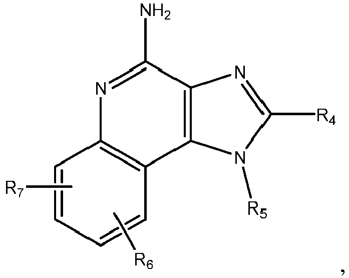 by reaction with a compound of the formula 3a or formula 3b: 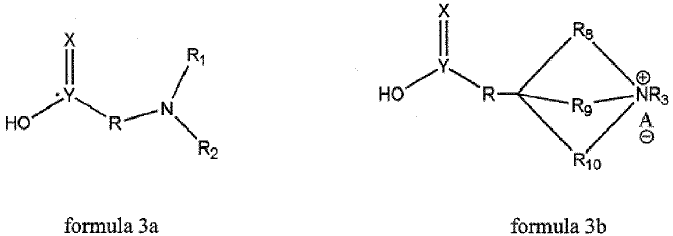 and a coupling reagent selected from N,N'Dicyclohexylcarbodiimide, N,N'-Diisopropylcarbodiimide, O-(Benzotriazol-1-yl)-N,N,N',N'tetramethyluronium tetrafluoroborate, O-(Benzotriazol-1-yl)-N,N,N',N'-tetramethyluronium hexafluorophosphate, and Benzotriazol-1-yl-oxy-tris(dimethylamino)phosphonium hexafluorophosphate, wherein,
R is a branched or straight chain, -(CH₂)ₙ-, wherein n=1, 2, 3, 4, 5, 6, 7, 8, 9, or 10 and any CH₂ may be replaced with O, S, CH=CH, or C≡C;
R₁ and R₂ are independently selected from H, C₁₋₁₅ alkyl, C₁₋₁₅ alkyloxyl, C₂₋₁₅ alkenyl, and C₁₋₁₅ alkynyl;
R₃ is H, C₁₋₁₅ alkyl, C₁₋₁₅ alkyloxy, C₂₋₁₅ alkenyl, C₁₋₁₅perfluoroalkyl, C₁₋₁₅ alkyl halide, or C₂₋₁₅ alkynyl;
X is O, NH, or S;
Y is C, S=O, or P-OR₁₁;
R₄ is selected from H, C₁₋₁₀ alkyl, C₁₋₁₀ hydroxylalkyl, and acyloxyalkyl, wherein the acyloxy moiety is C₂₋₅ alkanoyloxy or benzoyloxy and the alkyl moiety contains 1 to 8 carbon atoms, benzyl, (phenyl)ethyl and phenyl, wherein the benzyl, (phenyl)ethyl, or phenyl substituent is unsubstituted or substituted on the benzene ring by 1 or 2 moieties independently selected from C₁₋₁₅ alkyl, C₁₋₅ alkoxy, and halogen, with the proviso that if said benzene ring is substituted by 2 of said moieties, then said moieties together contain no more than 8 carbon atoms;
R₅ is selected from H, C₁₋₁₀ alkyl being unsubstituted or substituted by 1 or 2 moieties independently selected from hydroxy or cyclohexyl, benzyl, (phenyl)ethyl, and phenyl, said benzyl, (phenyl)ethyl, or phenyl substituent being unsubstituted or substituted on the benzene ring by 1 or 2 moieties independently selected from C₁₋₅ alkyl, C₁₋₅ alkoxy, and halogen, with the proviso that if said benzene ring is substituted by 2 of said moieties, then said moieties together contain no more than 8 carbon atoms;
R₆ is selected from H, C₁₋₅ alkyl, C₁₋₅ alkoxy, and halogen;
R₇ is selected from H, C₁₋₅ alkyl, C₁₋₅ alkoxy, and halogen;
R₈ is a branched or straight chain, -(CH₂)ₐ-, wherein a=1, 2, 3, 4, 5, 6, 7, 8, 9, or 10 and any CH₂ may be replaced with O, S, CH=CH, or C≡C;
R₉ is absent or a branched or straight chain, -(CH₂)_{b}-, wherein b= 1, 2, 3, 4, 5, 6, 7, 8, 9, or 10 and any CH₂ may be replaced with O, S, CH=CH, or C≡C;
R₁₀ is a branched or straight chain, -(CH₂)_{c}-, wherein c=1, 2, 3, 4, 5, 6, 7, 8, 9, or 10 and any CH₂ may be replaced with O, S, CH=CH, C≡C;
R₁₁ is H, C₁₋₁₅ alkyl, C₁₋₁₅ alkyloxy, C₂₋₁₅ alkenyl, C₁₋₁₅ perfluoroalkyl, C₁₋₁₅ alkyl halide, or C₂₋₁₅ alkynyl; and
A⁻ is a pharmaceutically acceptable negative ion.

10. A compound of formula (1) or formula (2) of any one of claims 1 to 8, or a composition comprising at least one compound of formula (1) or formula (2) as an active ingredient, which is in a form suitable for oral or transdermal administration, and which is for treating a 1H-imidazo[4,5-c]quinolin-4-amine-treatable condition in a human or animal.

11. Transdermal use of a compound of formula (1) or formula (2) according to any one of claims 1 to 8, for treating a 1H-imidazo[4,5-c]quinolin-4-amine-treatable condition in a human or animal, wherein the compound may optionally be in the form of a solution, a spray, a lotion, an ointment, an emulsion, or a gel.

12. A transdermal therapeutic application system comprising a compound of formula (1) or formula (2), according to any one of claims 1 to 8, as the active ingredient for treating a 1H-imidazo[4,5-c]quinolin-4-amine-treatable condition in a human or animal, wherein the system may optionally be a bandage or a patch comprising an active substance-containing matrix layer and an impermeable backing layer or an active substance reservoir that has a permeable bottom facing the skin.

13. The system according to claim 12, which controls the rate of release and enables the 1H-imidazo[4,5-c]quinolin-4-amine of the following formula 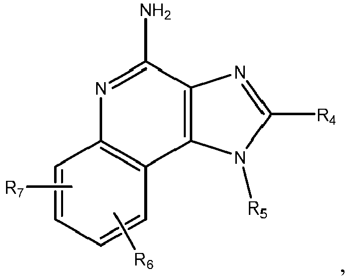 wherein
R₄ is selected from H, C₁₋₁₀ alkyl, C₁₋₁₀ hydroxylalkyl, and acyloxyalkyl, wherein the acyloxy moiety is C₂₋₅ alkanoyloxy or benzoyloxy and the alkyl moiety contains 1 to 8 carbon atoms, benzyl, (phenyl)ethyl and phenyl, wherein the benzyl, (phenyl)ethyl, or phenyl substituent is unsubstituted or substituted on the benzene ring by 1 or 2 moieties independently selected from C₁₋₁₅ alkyl, C₁₋₅ alkoxy, and halogen, with the proviso that if said benzene ring is substituted by 2 of said moieties, then said moieties together contain no more than 8 carbon atoms;
R₅ is selected from H, C₁₋₁₀ alkyl being unsubstituted or substituted by 1 or 2 moieties independently selected from hydroxy or cyclohexyl, benzyl, (phenyl)ethyl, and phenyl, said benzyl, (phenyl)ethyl, or phenyl substituent being unsubstituted or substituted on the benzene ring by 1 or 2 moieties independently selected from C₁₋₅ alkyl, C₁₋₅ alkoxy, and halogen, with the proviso that if said benzene ring is substituted by 2 of said moieties, then said moieties together contain no more than 8 carbon atoms;
R₆ is selected from H, C₁₋₅ alkyl, C₁₋₅ alkoxy, and halogen; and
R₇ is selected from H, C₁₋₅ alkyl, C₁₋₅ alkoxy, and halogen;
to reach an optimal therapeutic blood level to increase effectiveness and reduce the side effects of the 1H-imidazo[4,5-c]quinolin-4-amine of the above structure.

14. Oral or transdermal use of a compound of formula (1) or formula (2) according to any one of claims 1 to 8, or of a composition comprising at least one compound of formula (1) or formula (2) as an active ingredient, in combination with a non-steroidal anti-inflammatory drug or a prodrug thereof, for treating a 1H-imidazo[4,5-c]quinolin-4-amine treatable condition in a human or animal.

15. The use according to claim 14, wherein the compound or composition is in combination with diethylaminoethyl 2-(p-isobutylphenyl) propionate.AcOH, diethylaminoethyl 1-(ρ-chlorobenzoyl)-5-methoxy-2-methylindole 3-acetate.AcOH, diethylaminoethyl (Z)-5-fluoro-2-methyl-1-[(4-methylsulfinyl) phenylmethylene]-1H-indene-3-acetate.AcOH, diethylaminoethyl 1-methyl-5-(4-methylbenzoyl)-1H-pyrrole-2-acetate.AcOH, diethylaminoethyl 5-(4-Chlorobenzoyl)-1,4-dimethyl-1H-pyrrole-2-acetate.AcOH, or diethylaminoethyl 1,8-diethyl-1,3,4,9-tetrahydropyrano-[3,4-b]indole-1-acetate.AcOH.
